# EUROPEAN PATENT SPECIFICATION

(11) **EP 2 739 323 B1**
(45) Date of publication and mention of the grant of the patent: **26.09.2018**
(21) Application number: 12773382.2
(22) Date of filing: 31.07.2012
(51) Int. Cl.: A61L 27/06, A61L 27/54

(54) **PROCESS FOR PRODUCING MULTIFUNCTIONAL TITANIUM SURFACES FOR REDUCING THE RISK OF INFECTION AND INCREASED BONE INTEGRATION AND PRODUCT MADE THROUGH SUCH PROCESS**
VERFAHREN ZUR HERSTELLUNG VON MULTIFUNKTIONS- TITANOBERFLÄCHEN ZUR VERRINGERUNG DES RISIKOS VON INFEKTIONEN UND ERHÖHTER KNOCHENINTEGRATION SOWIE AUF DIESE WEISE HERGESTELLTES PRODUKT
PROCÉDÉ DE PRODUCTION DE SURFACES EN TITANE MULTIFONCTIONNELLES POUR RÉDUIRE LE RISQUE D'INFECTION ET ACCROÎTRE L'INTÉGRATION OSSEUSE ET PRODUIT OBTENU PAR CE PROCÉDÉ

(30) Priority: 01.08.2011 IT TO20110716
(43) Date of publication of application: 11.06.2014
(73) Proprietor: Politecnico Di Torino, 10129 Torino (IT)
(72) Inventor: SPRIANO, Silvia, I-10129 Torino (IT); VERNE', Enrica, I-10129 Torino (IT); FERRARIS, Sara, I-10129 Torino (IT)
(74) Representative: Garavelli, Paolo
(86) International application number: PCT/IT2012/000237
(87) International publication number: WO 2013/018118

(56) References cited:
- WO-A2-2009/047730
- US-A- 4 476 590
- DATABASE WPI Thomson Scientific, London, GB; AN 2003-633021 XP002670652, & KR 2003 0038631 A (LEE M H) 16 May 2003 (2003-05-16)

## Description

The present invention refers to a process for producing multifunctional titanium surfaces for reducing the risk of infection and for a quicker bone integration, and to a product made through such process.

The problem of infections in orthopedy is given by clinicians as "high medical need", whose occurrence is, for example, 1-2% in surgical operations for hip prostheses. Still higher is the occurrence of infections in mouth implants, such as for example the cases of perimplantitis. In both cases it is necessary to revise and remove the implant, with consequent patient's pain and strong discomfort.

It is known that some metals, such as silver, bismuth, copper and zinc, exert an antibacterial action, inhibiting both adhesion and proliferation of bacteria. In particular, silver has an antibacterial action which has long life and wide spectrum, even at very low concentrations (ppb), that is efficient also on bacterial species resisting to antibiotics (Staphylococcus aureus). It is also efficient as antifungal agent. For what is known so far, bacterial species do not develop resistance to the action of silver. Suitably dosed, silver has no genotoxic or cytotoxic effects (neither on osteoblasts nor on skin cells) neither local nor sistemic. What is stated above is valid essentially for silver in ionic form, while the mass metal does not need specific treatments in order to perform an antibacterial action (as described, for example in US 4476590), without exerting a cytotoxic action. A different case is the one of silver nano-particles, that are per se antibacterial.

Also copper has shown, in some studies, a good antibacterial efficacy, joined to a low toxicity.

There are some already known techniques for introducing these inorganic antibacterial agents on different biomaterials, like those disclosed in US4476590 and US2004129112A1, that provide for a ionic implant, those disclosed in US2004129112A1 that provide CVD/PVD techniques, those disclosed in US4476590 and US2004129112A1 that provide sputtering techniques, those disclosed in US4476590 that provide film evaporation techniques, those disclosed in US2009100792 that provide electrolitic anodisation/oxidation in silver-containing electrolytes, electro-deposition, formation of silver-containing nanotubes of sodium titanate or titanium oxide, like those disclosed in US2009093881A1, coating with Ag-containing sodium titanate, like those disclosed in CN101766540, sol-gel coatings, coatings with Ag-containing hydroxyapatite, plasma spray coatings.

In particular, the material produced according to CN101766540 has a coating made of sodium titanate and a surface with Na-composition gradient exploiting ionic exchange reactions that involve sodium.

Moreover, in the material produced according to US2010015193A1 the anti-bacterial agent is present in islands, while, in Journal of Clinical Neuroscience 18 (2011) 391-395, such agent is a coating that masks the underlying surface.

Moreover, the material produced according to US2009280156 has a non-permanent and biosoluble coating.

Moreover, Journal of Colloid and Interface Science 350 (2010) 402-408 discloses a process that uses a pre-prepared solution of Ag nano-particles.

International Journal of Nanomedicine 2010;5:261-267 instead discloses a process in which it is necessary to perform the surface silanisation followed by the coating step.

KR 2003 0038631 A discloses a surface treatment of a bioactive implant of titanium or titanium alloy comprising a surface treatment using a solution of hydrofluoric acid and nitric acid, a treatment using a mixed solution comprising hydrogen peroxide and metal chlorides (e.g. CuCl) and treatment by heat at 200-500 °C for 0.5-2 hours.

Therefore, object of the present invention is solving the above prior art problems by providing a process for producing multifunctional titanium surfaces for reducing the risk of infection and for a quicker bone integration that consists in exploiting the reaction between titanium, hydrogen peroxide and salt/oxide of Ag/Cu/Zn/Bi in a single stage.

Moreover, an object of the present invention is providing a process for producing multifunctional titanium surfaces for reducing the risk of infection and for a quicker bone integration that is less costly than those proposed by the prior art, since it does not require the use either of sophisticated apparatuses, or of corrosive solutions or the application of electric fields or long-lasting treatments, and with many stages, and has no risks of surface contamination, providing bioactive and anti-bacterial properties to the surface through a single process, with strong savings in terms both of times and of costs with respect to multi-stage solutions known in the art.

Another object of the present invention is providing a process for producing multifunctional titanium surfaces for reducing the risk of infection and for a quicker bone integration that, in the ternary system, has different characteristics from those that can be presumed taking into account the individual reactions of binary systems such hydrogen peroxide plus salt / Ag/Cu/Zn/Bi oxide and hydrogen peroxide/Ti, without using a pre-prepared solution of Ag nano-particles, and generating the anti-bacterial agent directly *in situ* on the surface.

Moreover, an object of the present invention is providing a process for producing multifunctional titanium surfaces for reducing the risk of infection and for a quicker bone integration that does not require the surface silanisation, nor coating steps, preserving the pre-existing surface roughness with respect to the treatment.

Moreover, an object of the present invention is providing a process for producing multifunctional titanium surfaces for reducing the risk of infection and for a quicker bone integration that allows modulating the amount of anti-bacterial agent introduces on the surface, in order to avoid that, in excessive does, it performs a cytotoxic action.

Another object of the present invention is providing a product that appears with a surface nano-porous layer made of titanium oxide containing the anti-bacterial agent as individual nano-particles homogeneously dispersed on the surface or as a ion.

Moreover, an object of the present invention is providing a product in which the distribution of the anti-bacterial agent is not present with islands nor it is a continuous coating.

Another object of the present invention is providing a product equipped with a single multifunctional surface that is bioactive, bone-inducing and anti-bacterial, permanent and non-biosoluble.

The above and other objects and advantages of the invention, as will appear from the following description, are obtained with a process for producing multifunctional titanium surfaces for reducing the risk of infection and for a quicker bone integration like the one claimed in claim 1.

Preferred embodiments and non-trivial variations of the present invention are the subject matter of the dependent claims.

It will be immediately obvious that numerous variations and modifications (for example related to shape, sizes, arrangements and parts with equivalent functionality) could be made to what is described, without departing from the scope of the invention as appears from the enclosed claims.

The present invention will be better described by some preferred embodiments thereof, provided as a non-limiting example.

In general, the process according to the present invention for producing a multifunctional surface provides for a surface modification of titanium or its alloys used, in particular, for arms and legs prostheses or teeth implants, with the purpose of introducing an inorganic antibacterial agent, that can be gradually released following the implant, inducing, at the same time, a bioactive behaviour and a multi-scale (macro-micro and nano) surface topography, adapted to promote the bone integration.

The process according to the present invention therefore comprises at least the following steps:
a) acid etching aimed to remove the native oxide present on the surface of the material made of titanium o a titanium alloy and to make this latter one micro-rough. Preferably, the step of acid etching is performed with that use of hydrofluoric acid diluted, for example, at a concentration from 0.5 M to 8 M, for a time included between 1 and 5 minutes;
b) preparation of a solution of hydrogen peroxide and of an anti-bacterial agent: preferably, the anti-bacterial agent, of the inorganic type, is a metal such as, for example, silver, bismuth, copper or zinc; still more preferably such metal is in ionic or powder form. Advantageously therefore the anti-bacterial agent is inserted during the oxidation in hydrogen peroxide, through the addition in solution of a salt or oxide thereof, that operates as precursor;
c) surface oxidation of the material made of titanium or a titanium alloy with such solution of hydrogen peroxide and anti-bacterial agent in order to enrich the surface of the above anti-bacterial agent, generate a characteristic surface topography (macro, micro and nano roughness) and hydroxyl groups linked to said surface.

The step of surface oxidation in step c) must be performed in such a way as to avoid the re-passivation (re-oxidation) of the surface between the steps of acid etching and oxidation.

Moreover, the process according to the present invention can comprise a step, included between step b) and step c), of surface oxidation of the material made of titanium or a titanium alloy with a solution of hydrogen peroxide whose concentration ranges between 15% and 70% in volume, preferably at a temperature included between 20°C and 80°C and for a time included between 30 and 400 minutes, more preferably between 30 and 225 minutes.

The developed layer of oxide has a high number of hydroxyles and a morphology sub-micrometric roughness and porosity.

Advantageously, the process according to the present invention allows exploiting the action of the hydrogen peroxide, in a single passage, for producing on one hand a layer of titanium oxide, that is bioactive and with a multi-scale topography and, on the other hand, the precipitation therein of an inorganic anti-bacterial agent. The process according to the present invention therefore allows exploiting a series of reactions, mutually sinergic, that occur in the ternary system hydrogen peroxide, titanium, Ag/Cu/Zn/Bi salt/oxide. During the reaction, the following simultaneously occur: oxidation of titanium, decomposition of hydrogen peroxide and deposition of the anti-bacterial agent on the titanium surface. Such reactions are mutually sinergic and linked: for example, the anti-bacterial agent also operates as catalyst of the decomposition of hydrogen peroxide and titanium catalyses the reduction of the anti-bacterial ion. Such reactions, made occur in a single stage, are performed under conditions of acid pH, that are different from what occurs for the individual reactions, made occur separately. Moreover, the process according to the present invention allows keeping the pre-existing roughness on the substrate. Described herein is a product made through the above-described process. In particular, the product, particularly suitable for orthopedy and teeth implants, comprises at least one substrate made of titanium, or an alloy thereof, with different surface roughness, with a surface comprising at least one nano-porous layer of titanium oxide containing the anti-bacterial inorganic agent (Ag and/or Cu and/or Zn and/or Bi) as individual nano-particles homogeneously dispersed on the surface or as ion. Therefore, it appears as multifunctional, namely as simultaneously bioactive (promoting the precipitation of hydroxyapatite in physiologic fluids), bone-inducing (having a multi-scale surface topography such as to stimulate the cells) and anti-bacterial (being able to reduce the risk of infections and the formation of biofilms in the implant site).

The product therefore has an optimised surface not only because it is bioactive and anti-bacterial, but also because it has suitable roughness, corrosion resistance, ionic release, surface topography and mechanical characteristics (hardness, fatigue and scratch resistance).

In particular, the product has (after immersion in a Simulated Physiologic Solution - SBF) in surface precipitates with the typical hydroxyapatite morphology, that are aggregates of small spherical particles. The EDS spectrum points out their qualitative chemical composition. The Ca/P ratio is 1.7, namely very near to the stoichiometric ratio for hydroxyapatite (1, 67). The sample surface not covered by particles is enriched in calcium and phosphorous following its immersion into SBF. This is an index of the diffused capability of the surface to adsorb these elements.

The chemically-treated samples (first in HF and then in H₂O₂) have been afterwards subjected to a step of heat treatment to stabilise the generated layer of oxide. The heat treatment can be performed in a temperature range included between 300°C and 600°C, in vacuum or air, or an in inert atmosphere, with a treatment lasting 1 to 8 hours. Surface morphology and bioactivity are kept following such heat treatment.

The hydroxyl groups have been demonstrated as thermally stable, since their presence (even if quantitatively modified) has been found after a prolonged storage period and after heat treatment.

Thickness and crystallinity of the layer of oxide can be modified, by changing temperature and atmosphere used in the heat treatment. A progressive increase is observed, as foreseen, in the peak related to anatase upon a temperature increase.

Moreover, the fatigue tests point out a low resistance decrease (2-15%) following the treatment; it can therefore be deemed that this type of surface modification does not meaningfully change the mechanical properties of the material.

Preferably, the process according to the invention can further comprise a step of functionalisation of the surface of titanium or an alloy thereof with bio-molecules that are bioactive from the biologic point of view, in particular natural or synthetic organic molecules involved in mineralisation mechanisms of the bone tissue (for example proteins of the extra-cellular matrix, peptides or enzymes involved in the adhesion/proliferation process, morpho-genetic proteins, growth factors, albumin, fibronectin, alkaline phosphatase and the like). The advantage of the surface functionalisation is the possibility of anchoring to the surface a controlled amount of bio-molecules, avoiding the risk of an excessive use, like the one following the local injection or the systemic administration.

Within the present invention, the bio-molecules of interest that are able of stimulating the cellular response are preferably covalently fixed to the surface of titanium or alloys thereof, previously made bioactive from the inorganic and antibacterial point of view. The direct covalent link has been chosen as anchoring mechanism, since it is more selective and allows a better adhesion with respect to the simple absorption or the release of carrier capable of be re-absorbed.

The adopted functionalisation process avoids the silanisation or use of spacer molecules that can be toxic (such as glutaraldehyde). In particular, in the adopted functionalisation step, the presence of hydroxyl groups on the surface of the treated metal is fundamentally important, since such groups are the reactive functional group useful for reactions that bring about the surface functionalisation.

In particular, for this surface modification, the step of functionalisation comprises the substeps of:
- activation of the surface of titanium or alloys thereof, previously treated according to steps a), c) and optionally according to the heat treatment step, with an organic chloride, preferably with trifluoroethansulphonil chloride, pure or in solution; and
- incubation of the activated surfaces with a solution containing a bio-molecule.

Preferably, the step of activation with trifluoroethansulphonil chloride is performed at a reaction temperature from 20°C to 50°C, with times from 10 minutes to 72 hours. Preferred conditions are a temperature of 37°C for 48 hours.

Moreover, preferably, the incubation step is performed by exposing the activated surface to the above solution having a concentration included between 2 and 40 mg/ml, for a time from 0.5 to 72 hours, at a temperature included between 0°C and 50°C.

Moreover, the bio-molecule can be a natural, recombinant or synthetic bio-molecule, chosen among bio-adhesives, cellular adhesion factors, biopolymers, blood proteins, enzymes, proteins or bio-molecules of the extra-cellular matrix, growth factors, hormones, pepticides, deoxiribonucleic acids (DNA), ribonucleic acids (RNA), receptors, enzymatic inhibitors, drugs, biologically active anions and cations, peptides, morphogenetic proteins, adhenosin monophosphate (AMP), adhenosin diphosphate (ADP), adhenosin triphosphate (ATP), albumin, fibronectin, alkaline phosphatase or a combination thereof.

In a specific example, the biologic functionalisation has been performed by using the alkaline phosphatase (ALP) enzyme, that is involved in the mineralisation processes of hard tissues; different studies in literature suggest that its local application promotes the growth and mineralisation of the damaged bone tissue. Moreover, ALP appears as a good model molecule, since it is rather simple to locate. It is however meant that the step of functionalisation with bio-molecules is not limited to the specific example described, being it possible to use in general proteins of the extra-cellular matrix or enzymes involved in the cellular adhesion/proliferation process, as mentioned above.

The presence of a relevant amount of hydroxyl groups on the surface is fundamental for the material response to this second step.

The final step provides for the enzymatic anchorage to the treated surfaces. For such purpose, the bio-molecule of interest is placed in a solution, preferably in PBS, with a concentration preferably included between 2 and 40 mg/ml, for a time from 0.5 to 72 hours, at a temperature preferably included between 0°C and 50°C.

In a specific example, a solution of ALP in PBS has been used, with a concentration of 5 mg/ml for an incubation time of 24 hours at 4°C.

A direct anchorage of the molecule to the titanium surface has been obtained, since trifluoroethansulphonil chloride behaves as a good outgoing group.

The enzyme activity following its anchorage onto the surface has been determined through a visible UV analysis, following the reaction with paranitrophenilphosphate. This reaction produces paranitrophenol that, in an alkaline environment, has a yellow colour. The yellow intensity, measured spectrophotometrically, can be related to the amount of ALP present on the surface.

## Claims

1. Process for producing a multi-functional surface, in particular for reducing the risk of infection and for a quicker bone integration, through a modification treatment of the surface made of titanium or a titanium alloy, said multi-functional surface comprising at least one substrate of titanium, or an alloy thereof, with a surface comprising at least one nano-porous layer of titanium oxide containing an inorganic anti-bacterial agent as individual nano-particles homogeneously dispersed on the surface, said process comprising the steps of:
a) acid etching for removing the native oxide present on said surface, said acid etching step being performed with hydrofluoric acid only;
b) preparation of a solution of hydrogen peroxide and of the anti-bacterial agent; and
c) surface oxidation of said titanium or said titanium alloy with said solution of hydrogen peroxide and of the anti-bacterial agent, in order to enrich the surface of said anti-bacterial agent, generate a characteristic surface topography and generate hydroxyl groups linked to said surface, wherein said surface oxidation step c) is performed in order to avoid the surface re-passivation between said steps of acid etching a) and surface oxidation c),
wherein said solution of hydrogen peroxide has a concentration between 15% and 70% in volume, at a temperature included between 20°C and 80°C and for a time included between 30 and 400 min, preferably for a time between 30 and 225 minutes; and
wherein said process is performed under acid pH conditions.

2. Process according to claim 1, **characterised in that**, between step b) and step c), it comprises a step d) of surface oxidation of said material made of titanium or said titanium alloy with a solution of hydrogen peroxide.

3. Process according to claim 1, **characterised in that** said anti-bacterial agent is inorganic.

4. Process according to claim 1, **characterised in that** said anti-bacterial agent is a metal.

5. Process according to claim 4, **characterised in that** said metal is silver, bismuth, copper or zinc.

6. Process according to claim 4, **characterised in that** said metal is in ionic or powder form.

7. Process according to claim 1, **characterised in that** it comprises, after said step c), an heat treatment step for stabilising the generated layer of oxide.

8. Process according to claim 7, **characterised in that** said heat treatment step is performed with an heat treatment at a temperature included between 300°C and 600°C, with times from 1 to 8 hours in air, vacuum or inert atmosphere.

9. Process according to any one of the previous claims, **characterised in that** it comprises a step of functionalisation of said surface of titanium or of titanium alloy with bio-molecules containing biologic bioactivity.

10. Process according to claim 9, **characterised in that** said functionalisation step comprises the substeps of:
- activation of the surface of titanium or alloys thereof, previously treated according to steps a), c) and optionally according to the heat treatment step with an organic chloride, said activation being performed with trifluoroethansulphonil chloride, pure or in solution; and
- incubation of the activated surfaces with a solution containing a bio-molecule.

11. Process according to claim 10, **characterised in that** said step of activation with trifluoroethansulphonil chloride is performed at a reaction temperature from 20°C to 50°C, with times from 10 minutes to 72 hours.

12. Process according to claim 10 or 11, **characterised in that** said incubation step is performed by exposing said activated surface to said solution having a concentration included between 2 and 40 mg/ml, for a time from 0.5 to 72 hours, at a temperature included between 0°C and 50°C.

13. Process according to any one of the previous claims, **characterised in that** said bio-molecule is chosen among natural or synthetic organic molecules involved in mineralisation mechanisms of the bone tissue.

14. Process according to any one of claims 9 to 13, **characterised in that** said bio-molecule is a natural, recombinant or synthetic bio-molecule, bio-adhesives, cellular adhesion factors, biopolymers, blood proteins, enzymes, proteins or bio-molecules of the extra-cellular matrix, growth factors, hormones, pepticides, deoxiribonucleic acids (DNA), ribonucleic acids (RNA), receptors, enzymatic inhibitors, drugs, biologically active anions and cations, peptides, morphogenetic proteins, adhenosin monophosphate (AMP), adhenosin diphosphate (ADP), adhenosin triphosphate (ATP), albumin, fibronectin, alkaline phosphatase or a combination thereof.

## Patentansprüche

1. Verfahren für die Produktion einer multifunktionalen Oberfläche, insbesondere zur Reduzierung des Infektionsrisikos und für eine schnelle Osteointegration durch eine Behandlung zur Änderung der Oberfläche aus Titan oder Titanlegierung, die genannte multifunktionale Oberfläche umfasst mindestens ein Titansubstrat oder eine seiner Legierungen mit einer Oberfläche, die mindestens eine nanoporöse Schicht aus Titanoxid enthält, welche einen anorganischen antibakteriellen Wirkstoff als einzelne Nanopartikel enthält, die homogen auf der Oberfläche zerstreut sind, das genannte Verfahren schließt folgende Phasen ein:
a) Säureangriff zur Entfernung des nativen Sauerstoffs auf der genannten Oberfläche, die Säureangriffsphase wird nur mit Fluorwasserstoffsäure ausgeführt;
b) Zubereitung einer Lösung aus Hydrogenperoxid und dem antibakteriellen Wirkstoff; und
c) Oberflächenoxidation des genannten Titans oder der genannten Titanlegierung mit der genannten Lösung aus Hydrogenperoxid und dem antibakteriellen Wirkstoff, um die Oberfläche des o. g. antibakteriellen Wirkstoffs anzureichern, eine charakteristische Oberflächentopografie und an die genannte Oberfläche gebundene Hydroxylgruppen zu erzeugen, in der die genannte Phase c) der Oberflächenoxidation so ausgeführt wird, dass die Repassivierung der Oberfläche in den genannten Säureangriffs- a) und Oxidationsphasen c) vermieden wird,
in der die genannte Hydrogenperoxidlösung eine Volumenkonzentration von 15% bis 70% bei einer Temperatur zwischen 20 °C und 80 °C für eine Zeit zwischen 30 und 400 min, vorzugsweise für eine Zeit zwischen 30 und 225 Minuten, hat; und
in der das genannte Verfahren mit saurem pH-Wert ausgeführt wird.

2. Verfahren gemäß Patentanspruch 1, das **dadurch gekennzeichnet ist, dass** es zwischen der Phase b) und der Phase c) eine Phase d) der Oberflächenoxidation des genannten Titanmaterials oder der genannten Titanlösung mit einer Hydrogenperoxidlösung enthält.

3. Verfahren gemäß Patentanspruch 1, das **dadurch gekennzeichnet ist, dass** der genannte antibakterielle Wirkstoff anorganisch ist.

4. Verfahren gemäß Patentanspruch 1, das **dadurch gekennzeichnet ist, dass** der genannte antibakterielle Wirkstoff ein Metall ist.

5. Verfahren gemäß Patentanspruch 4, das **dadurch gekennzeichnet ist, dass** das genannte Metall Silber, Wismut, Kupfer oder Zink ist.

6. Verfahren gemäß Patentanspruch 4, das **dadurch gekennzeichnet ist, dass** das genannte Metall in Ionen- oder Pulverform ist.

7. Verfahren gemäß Patentanspruch 1, das **dadurch gekennzeichnet ist, dass** es nach der genannten Phase c) eine Phase der Wärmebehandlung zur Stabilisierung der erzeugten Oxidschicht enthält.

8. Verfahren gemäß Patentanspruch 7, das **dadurch gekennzeichnet ist, dass** die genannte Phase der Wärmebehandlung mit Wärmebehandlung bei einer Temperatur von 300 °C bis 600 °C für eine Zeit von 1 bis 8 Stunden in Luft, Vakuum oder inerter Atmosphäre durchgeführt wird.

9. Verfahren gemäß einem beliebigen der vorhergehenden Patentansprüche, das **dadurch gekennzeichnet ist, dass** es eine Funktionalisierungsphase der genannten Oberfläche aus Titan oder der genannten Titanlegierung mit Biomolekülen enthält, die eine biologische Aktivität enthalten.

10. Verfahren gemäß Patentanspruch 9, das **dadurch gekennzeichnet ist, dass** die genannte Funktionalisierungsphase folgende Unterphasen einschließt:
- Aktivierung der Oberfläche aus Titan oder seinen Legierungen, die vorhergehend gemäß den Phasen a), c) und optional gemäß der Phase der Wärmebehandlung mit einem organischen Chlorid behandelt wird, die genannte Aktivierung wird mit Trifluorethansulfonylchlorid, rein oder in Lösung, ausgeführt; und
- Inkubation der aktivierten Oberflächen mit einer Lösung, die ein Biomolekül enthält.

11. Verfahren gemäß Patentanspruch 10, das **dadurch gekennzeichnet ist, dass** die genannte Aktivierungsphase mit Trifluorethansulfonylchlorid bei einer Reaktionstemperatur von 20 °C bis 50 °C mit Zeiten von 10 Minuten bis 72 Stunden ausgeführt wird.

12. Verfahren gemäß Patentanspruch 10 oder 11, das **dadurch gekennzeichnet ist, dass** die genannte Inkubationsphase ausgeführt wird, indem die aktivierte Oberfläche der Lösung, die eine Konzentration von 2 bis 40 mg/ml hat, für eine Zeit von 0,5 bis 72 Stunden einer Temperatur zwischen 0 °C und 50 °C ausgesetzt wird.

13. Verfahren gemäß einem beliebigen der vorhergehenden Patentansprüche, das **dadurch gekennzeichnet ist, dass** das genannte Biomolekül unter natürlichen organischen oder synthetischen Molekülen gewählt wird, die von den Mechanismen der Mineralisierung des Knochengewebes betroffen sind.

14. Verfahren gemäß einem beliebigen der vorhergehenden Patentansprüche von 9 bis 13, das **dadurch gekennzeichnet ist, dass** das genannte Biomolekül ein natürliches, rekombinantes oder synthetisches Biomolekül ist, ausgewählt aus Bioklebstoffen, zellulären Adhäsionsfaktoren, Biopolymeren, Blutproteinen, Enzymen, Proteinen oder Biomolekülen aus der extrazellulären Matrix, Wachstumsfaktoren, Hormonen, Peptiden, Deoxyribonukleinsäuren (DNA), Ribonukleinsäuren (RNA), Rezeptoren, enzymatischen Inhibitoren, Arzneistoffen, biologisch aktiven Anionen und Kationen, Peptiden, morphogenetischen Proteinen, Adenosinmonophosphat (AMP), Adenosindiphosphat (ADP), Adenosintriphosphat (ATP), Albumin, Fibronectin, alkalischer Phosphatase und Kombinationen der vorstehend genannten Moleküle.

## Revendications

1. Procédé de préparation d'une surface multifonction, en particulier pour la réduction du risque d'infection et pour une plus rapide ostéo-intégration, par l'intermédiaire d'un traitement de modification de la surface en titane ou en alliage de titane ; cette surface multifonction comprend au moins un substrat de titane, ou l'un de ses alliages, avec une surface comprenant au moins une couche nano-poreuse d'oxyde de titane contenant un agent antibactérien inorganique comme nanoparticules individuelles dispersées de manière homogène sur la surface, la procédure comprend les étapes suivantes :
a) une attaque acide pour éliminer l'oxyde natif présent sur la surface, cette étape d'attaque acide est exécutée uniquement avec de l'acide fluorhydrique ;
b) une préparation d'une solution composée de peroxyde d'hydrogène et d'agent antibactérien ; et
c) une oxydation superficielle du titane ou de l'alliage de titane avec la solution composée de peroxyde d'hydrogène et d'agent antibactérien, dans le but d'enrichir la surface de l'agent antibactérien, de créer une topographie superficielle caractéristique et de générer des groupements hydroxyles liées à cette surface, où l'étape c) d'oxydation superficielle est exécutée de sorte à éviter la repassivation de la surface entre les étapes d'attaque acide a) et d'oxydation c),
où la solution de peroxyde d'hydrogène a une concentration entre 15% et 70% en volume, à une température de 20°C à 80°C, pendant une durée comprise entre 30 et 400 min (la durée préférable est de 30 à 225 minutes) ;
et où le procédé est exécuté en conditions de pH acide.

2. Procédé, selon la revendication 1, **caractérisé en ce que**, entre l'étape b) et l'étape c), elle comprend une étape d) d'oxydation superficielle du matériau en titane ou en alliage de titane avec une solution de peroxyde d'hydrogène.

3. Procédé, selon la revendication 1, **caractérisé en ce que** l'agent antibactérien est inorganique.

4. Procédé, selon la revendication 1, **caractérisé en ce que** l'agent antibactérien est un métal.

5. Procédé, selon la revendication 4, **caractérisé en ce que** le métal est argent, bismuth, cuivre ou zinc.

6. Procédé, selon la revendication 4, **caractérisé en ce que** le métal est sous forme ionique ou en poudre.

7. Procédé, selon la revendication 1, **caractérisé en ce qu'**elle comprend, après l'étape c), une étape de traitement thermique pour la stabilisation de la couche d'oxyde engendrée.

8. Procédé, selon la revendication 7, **caractérisé en ce que** l'étape de traitement thermique est effectuée avec un traitement thermique à température de 300°C à 600°C, pendant une durée comprise entre 1 et 8 heures dans l'air, le vide ou en atmosphère inerte.

9. Procédé selon l'une des revendications précédentes, **caractérisé en ce qu'**il comprend une étape de fonctionnalisation de la surface en titane ou en alliage de titane avec des biomolécules contenant une bio-activité biologique.

10. Procédé, selon la revendication 9, **caractérisé en ce que** l'étape de fonctionnalisation comprend les sous-étapes de :
- activation de la surface de titane ou de ses alliages, traitée précédemment selon les étapes a), c) et, en option, selon l'étape de traitement thermique avec un chlorure organique, cette activation est exécutée avec du chlorure de trifluoroéthanesulfonyle, pur ou en solution ; et
- incubation des surfaces activées avec une solution contenant une biomolécule.

11. Procédé, selon la revendication 10, **caractérisé en ce que** l'étape d'activation avec du chlorure de trifluoroéthanesulfonyle est effectuée à une température de réaction de 20°C à 50°C, pendant une durée comprise entre 10 minutes et 72 heures.

12. Procédé, selon la revendication 10 ou 11, **caractérisé en ce que** l'étape d'incubation est réalisée en exposant la surface activée à la solution ayant une concentration comprise entre 2 et 40 mg/ml, pendant une durée comprise entre 0,5 et 72 heures, à une température de 0°C à 50°C.

13. Procédé selon l'une des revendications précédentes, **caractérisé en ce que** la biomolécule est choisie parmi les molécules organiques naturelles ou synthétiques impliquées dans les processus de minéralisation osseuse.

14. Procédé selon l'une des revendications de 9 à 13, **caractérisé en ce que** la biomolécule est une biomolécule naturelle, recombinée ou synthétique, choisie parmi choisie parmi les bio-adhésifs, les facteurs d'adhésion cellulaire, les biopolymères, les protéines du sang, les enzymes, les protéines ou les biomolécules provenant de la matrice extracellulaire, les facteurs de croissance, les hormones, les peptides, l'acide désoxyribonucléique (ADN), l'acide ribonucléique (ARN), les récepteurs, les inhibiteurs enzymatiques, les médicaments, les anions et les cations biologiquement actifs, les peptides, les protéines morphogénétiques, l'adénosine monophosphate (AMP), l'adénosine diphosphate (ADP), l'adénosine triphosphate (ATP), l'albumine, la fibronectine, la phosphatase alcaline et les combinaisons des molécules précédemment citées.
